## Europäisches Patentamt
## European Patent Office
## Office européen des brevets

(11) Publication number: **0 151 203**
**B1**

(12) **EUROPEAN PATENT SPECIFICATION**

(45) Date of publication of patent specification: **16.08.89**

(51) Int. Cl.⁴: **A 61 K 7/30**

(21) Application number: **84101303.0**

(22) Date of filing: **08.02.84**

(54) Anti-plaque denture cleansing tablet.

(43) Date of publication of application:
**14.08.85 Bulletin 85/33**

(45) Publication of the grant of the patent:
**16.08.89 Bulletin 89/33**

(84) Designated Contracting States:
**AT BE CH DE FR GB IT LI LU NL SE**

(56) References cited:
**EP-A-0 028 005**
**DE-B-2 452 583**

(73) Proprietor: **Richardson GmbH**
**H.-S.-Richardson-Strasse Postfach 1661**
**D-6080 Gross-Gerau (DE)**

(72) Inventor: **Levy, Alan Abraham**
**12 Bentley Way**
**Stanmore Middlesex (GB)**
Inventor: **Brudney, Norman**
**119 Avenue Victor Hugo**
**F-75116 Paris (FR)**

(74) Representative: **Vossius & Partner**
**Siebertstrasse 4 P.O. Box 86 07 67**
**D-8000 München 86 (DE)**

The file contains technical information
submitted after the application was filed and
not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This invention relates to a two phase denture cleansing tablet possessing anti-plaque activity as well as good cleansing, bleaching, disinfecting and antibacterial properties.

Known compositions for cleaning dentures are generally of two types:

a. alkaline compositions containing an oxidising agent which is effective in bleaching the stains and films which form on dentures but which do not readily dissolve tartar deposits;

b. acidic compositions which are effective in removing tartar deposits but which, if not properly rinsed, tend to leave an acidic after taste on the dentures and may cause corrosion in exposed metallic parts.

It has also been proposed, for example, in U.S. Patent 3,997,459 of K. Bogie et al to provide a solid denture cleaning composition comprising a tartar dissolving amount of an acidic component which dissolves to provide an acidic solution when the composition is mixed with water and a neutralizing composition is mixed with water and a neutralizing component having a protective coating, in an amount such that, when the composition is mixed with water the protective coating is so affected that the neutralizing component is released over a period of time to adjust the initially acidic solution to a pH of more than 5.5.

Also in German Offenlegungsschrift No. 2,357,720 and U.S. Patent 4,256,599 of Werner Krisp et al there are disclosed two-layered denture cleansing tablets with different dissolution rates for improved cleaning of dentures. Each layer of such tablets provides a pH in solution upon dissolution of the layers of about 6.5 to 7.0.

While each of the above mentioned denture cleansing tablets provides dentures cleansing properties sufficient for the cleansing of dentures, none of said tablets provides anti-plaque properties nor has there been any denture cleansing tablet made available with anti-plaque activity.

EP—A—0 028 005 discloses a two-layer denture cleansing tablet with a first layer having a faster dissolution rate producing a weakly alkaline solution while a second slower dissolving layer produces an acidic solution. The tablet may also contain cetyl ammonium bromide (CAB) in either of the layers. This compound is not suitable in carrying out the present invention because, once attached to the plaque or to the denture material, it remains attached without being released to the culture medium of the surrounding plaque growth area. Furthermore, staining of the dentures cannot be excluded when using CAB, and its antimicrobial activity is lower than compared with cetyl pyridinium chloride (CPC) which is used in the present invention.

It has also been known that cetylpyridinium chloride has anti-plaque activity in mouthwash compositions, such as for example, as disclosed by A. R. Volpe et al., J. Dental Research, 48, 832—841 (1969); J. K. Lem et al., Caries Research, 440—442 (1982); and J. D. Holbecke et al., Australian Dental Journal, 23(4), 328—331, August 1978. Although cetylpyridinium chloride was found to possess anti-plaque activity in mouthwashes attempts to obtain anti-plaque activity for cetylpyridinium chloride in solid forms, such as lozenges, have not been successful. For example, G. P. Barnes et al, J. Preventive Denstistry, 2(5) 2—4, 6 Sept.-Oct., 1975 reported that neither the use of one cetylpyridinium chloride lozenge nor the use of three such lozenges per day resulted in a significant change in clinical plaque scores and that the different dosage levels of cetylpyridinium chloride used in the study were equally ineffective in reducing existing plaque accumulations and in inhibiting new plaque formation. Thus it has not heretofore been considered possible to obtain the antiplaque properties of cetylpyridinium chloride in solid dosage form and particularly in a denture cleansing tablet.

It is therefore highly desirable that a denture cleansing composition be provided such that a denture cleansing tablet possessing anti-plaque activity as well as good cleansing, bleaching, disinfecting and antibacterial activity be provided. Attempts to do so before applicants' invention have not proved successful.

It has now been discovered that a denture cleansing tablet possessing good anti-plaque activity as well as good cleansing bleaching disinfecting and antibacterial activity can be obtained by providing a two-layered denture cleansing tablet having

(A) a first, faster dissolution, alkaline layer containing active oxygen cleansing and disinfecting compounds which dissolves in water within about two minutes to produce a solution having a pH of about 9.0, and

(B) a second, slower dissolution, acid layer containing an anti-plaque effective amount of quaternary ammonium anti-plaque compound selected from

tetradecyl trimethylammonium bromide;

1-hexadecyl pyridinium chloride;

benzyl, dimethyl (2-(2-(4-(1,1,3,3-tetra methyl butyl)phenoxylethoxy)ethyl)ammonium chloride;

a mixture of alkyl benzyl dimethylammonium chlorides;

dodecyl dimethyl (2-phenoxyethyl)ammonium bromide;

1,1-decamethylene bis (4-amino-2-methylquinolinium chloride); and

dimethyl tetradecyl benzyl ammonium chloride,

and acidic compounds which dissolves in water in a period up to about nine minutes and together with the alkaline layer produces a solution having a pH of about 3.0 ± 0.5 thereby permitting deposition of an anti-plaque effective amount of the quaternary ammonium compound on dentures.

With such a two-phase tablet the first phase cleanses and disinfects under alkaline conditions for a period of up to about two minutes and the second phase cleanses under acidic conditions and removes some calcified deposits (tartar) on the dentures as well as further disinfecting and coating the dentures with an anti-plaque effective amount of the anti-plaque agent cetylpyridinium chloride (= 1-hexadecyl pyridinium chloride). Generally the total dissolution time for the two layers would preferably be less than about nine minutes.

A two-phase tablet is provided which in use produces a cleansing solution initially producing a pH of about 9.0 and which then swings to a pH of about 3.0 ± 0.5 upon dissolution of the second, acid layer of the tablet. The denture cleansing tablet producing such a pH swing is provided by a two-layered tablet having different dissolution rates for the two layers which is accomplished by the selection of ingredients in each layer and by adjustment of the hardness of the tablet to a hardness of between about 6 and 10 kp (58.86 and 98.1 N).

The composition of the first, faster dissolution, alkaline layer of a tablet of the invention comprises on a dry weight basis, based on 100 weight percent total dry weight first layer, from 10 to 25% sodium bicarbonate, 4 to 15% sodium tripolyphosphate, 5 to 40% sodium perborate and 10 to 50% potassium monopersulphate.

The composition of the second, slower dissolution, acid layer of a tablet of this invention comprises on a dry weight basis, based on 100 weight percent total dry weight second layer, from 10 to 60% sulphamic acid, 10 to 90% potassium monopersulphate and/or citric acid, and up to 10% quaternary ammonium anti-plaque compound selected from the group disclosed hereinabove, such as cetylpyridinium chloride. A mixture of potassium monopersulphate or citric acid may also be used including other acids such as bisulphate.

Such tablets are useful for the self-acting cleansing of dentures in an aqueous medium whereby the dentures are cleaned, bleached and disinfected at a pH of about 9 from the alkaline layer and upon dissolution of the second acid layer the pH of the solution swings to about 3.0 and tartar is removed and cetylpyridinium chloride is deposited on the dentures and this deposition inhibits built-up of plaque on the dentures.

The monopersulphate employed in the invention is preferably a 50% active form known as Caroat® and sold by E.I. du Pont de Nemours Co. and is a 2:1:1 mixture of monopersulphate, potassium sulphate and potassium bisulphate. This mixture has an active oxygen content of approximately 4.5%.

The first, faster dissolution alkaline layer evidences maximum decomposition at about pH 9, that is, a decomposition reaction of the monopersulphate and perborate ingredients to give off active oxygen to bleach and disinfect the dentures as well as providing a cleansing action. This decomposition reaction stops when pH of the solution is acidic. At this high pH the perborate is most active by providing a hydrogen peroxide source (active oxygen) for bleaching and disinfecting the dentures. The sodium tripolyphosphate component is a water softener/sequestering agent as well as a detergent which dissolves rapidly and removes loose plaque and food debris from the dentures.

The sodium bicarbonate component is present to react with the potassium bisulphate which is present in the commercial grade of potassium monopersulphate to improve effervescence due to carbon dioxide production and hence increase the rate of dissolution of the layer. Sodium carbonate may optionally be present in the composition in an amount of from 0 to about 15% dry weight basis. The sodium carbonate and sodium tripolyphosphate ingredients are responsible for giving the high alkaline pH during the two minute dissolution of the alkaline layer. Too much phosphate and carbonate with a corresponding decrease in the bicarbonate content would give an unacceptably high pH. The rate of effervescence and dissolution would decrease, particularly if levels of perborate and monopersulphate are reduced as well to accommodate the increase in the alkaline ingredients. The presence of sodium carbonate also is useful in improving the stability of the product.

If the bicarbonate content is increased significantly so as to improve the tabletting properties at the expense of carbonate and tripolyphosphate which are more difficult to tablet then the pH swing and final pH would be dramatically affected, for example, pH will not rise to 8 or 9 and the final pH would be significantly lower than the desired pH of 3.0 ± 0.5.

Of course other optional ingredients can be present in the first, alkaline layer provided the ingredients are not detrimental to the overall activity of the two-phase tablet and do not materially affect the required pH swing of from about 9 to about 3.0 ± 0.5. For example, a complexing agent for a softening and demineralizing effect as well as a tartar-dissolving effect and detergency action can be employed. Any suitable complexing agent, such as for example, an ethylene diamine tetraacetate, such as ethylene diamine tetraacetic acid or its sodium salts, can be employed. Such an agent also improves the stability of any dye or colorant present but since it has poor tabletting properties its concentration is kept to a minimum, for example, from 0 to 5% dry weight basis, preferably about 3%.

Binding agents to stop lamination and/or capping of the tablet may be employed; such as for example, powdered nonionic surfactants, such as polyethanoxy alkyl ether sold as EMPILAN® KM50 and the like, or anionic surfactants such as dodecylbenzene sulphate, sodium lauryl sulphate or the like. The agents also improve the foaming properties and the dispersion of any precipitate formed in the solution. Selection of these ingredients, which are also detergents, must consider their effect on sticking on tablet punches and on the hardness of the tablet which could increase causing a decrease in the rate of dissolution. The amount of such agents will generally be from 0 to 5% dry weight basis, preferably about 1%. Especially preferred for use in this connection is ricinylmonoethanolamide sulphosuccinate disodium salt sold under the trademark REWO—DERM® S1333 from the firm REWO, Federal Republic of Germany and a mixture of hardened triglycerides material being available in the Federal Republic of Germany under the trademark BOESON® VP 60. Polyethylene glycol, such as for example, PEG 20,000 may also be employed. Generally up to about 3% dry weight basis of the sulfosuccinate and up to about 5% hardended triglyceride may be employed, preferably about 1% of each.

Flavoring agents such as peppermint or other aromatics may be added in an amount of from 0 to about 1.5%, preferably about 0.3%. While any suitable dye or colorant could be added, it is preferred that the alkaline layer be white and that a suitable dye or colorant be employed in the acid layer to evidence the dissolution of the acid layer and the pH swing of the cleansing solution.

The second, slower dissolution, acid layer generally dissolves in 6 to 8 minutes and this phase interacts with the alkaline phase to give a final solution pH of about 3.0 ± 0.5. This is important since it has been found that deposition of cetylpyridinium chloride and hence antiplaque activity is maximized under acidic conditions. Moreover, any significant increase in the final pH will also decrease the removal of calcium deposit and any acid cleansing properties.

In the second acid layer sulphamic acid and potassium monopersulphate (Caroat®) are the main ingredients used to achieve the low pH required for acid cleansing and deposition of cetylpyridinium chloride onto the dentures. Additionally it is possible to employ some sodium bisulphate for this acid lowering function since it is easier to tablet than sulphamic acid. However, sodium bisulphate is notorious for its corrosive and hygroscopic properties. Moreover, increasing the sodium bisulphate content to improve the tabletting properties decreases the stability of the acid layer. Thus sodium bisulphate can be employed in the acid layer in an amount of from 0 to 20%, preferably about 5%, on a dry weight basis, along with sulphamic acid.

The potassium monopersulphate present in the acid phase provides bleaching and antibacterial properties, maintains acidity and acts as a tabletting aid. In addition to its anti-plaque activity cetylpyridinium chloride also has antibacterial properties in the tablet of this invention.

Although the preferred quaternary ammonium anti-plaque compound is cetyl pyridinium chloride, other such quaternary compounds can be employed in the tablets of this invention, namely, the following quaternary compounds:

tetradecyl trimethylammonium bromide;

1-hexadecyl (i.e. cetyl) pyridinium chloride;

benzyl dimethyl (2-(2-[4-(1,1,3,3-tetra methyl butyl)phenoxy]ethoxy)ethyl)ammonium chloride;

a mixture of alkyl benzyl dimethylammonium chlorides;

dodecyl dimethyl (2-phenoxyethyl)ammonium bromide;

1,1-decamethylene bis (4-amino-2-methylquinolinium chloride); or

dimethyl tetradecyl benzyl ammonium chloride.

Other optional or desirable ingredients may also be added to the second acid layer provided they are not detrimental to the overall activity of the two-phase tablet and do not materially affect the required pH swing of from 9 to 3.0 ± 0.5. For example, as in the first layer, a complexing agent for a softening and demineralizing effect as well as a tartar-dissolving effect and detergency action can be employed. The same suitable complexing agents as in the alkaline layer may be employed, preferably the tetrasodium salts of ethylene diamine tetraacetic acid, in an amount of from 0 to 10%, dry weight basis, preferably about 2%.

As with the alkaline layer, sodium carbonate in an amount of from 0 to 10%, preferably about 2%, dry weight basis can be employed for the same general reasons as employed in the alkaline phase. However, the amount of carbonate in the acid layer is low so as to reduce the rate of effervescence and hence dissolution of this layer.

Disodium pyrophosphate employed in the acid phase has softening and cleansing properties similar to the tripolyphosphate in the alkaline phase. Raising or lowering the amount of the ingredient does not affect the pH profile because of its neutral pH and it is therefore employed as an excipient for adjusting the formulation in an amount of from about 0 to about 15% dry weight basis.

A further preferred embodiment of the inventive cleansing tablet is distinguished by virtue of the fact that the second layer contains 0.1 to 3%, preferably about 0.5%, by weight on a dry weight basis, silicone dioxide which has been manufactured by means of the pyrolysis of silicon tetrachloride in an oxyhydrogen flame. It is through this silicone dioxide which is available commercially under the trade name "Aerosil"®, that an effective protection of the composition of the second layer against hygroscopicity is achieved. The silicone dioxide has lubricating properties and maintains the free flowing properties of the powder.

Binding agents to stop lamination and/or capping of the tablet may be employed, such as, for example, powdered nonionic surfactants such as, polyethanoxy alkyl ether, sold as EMPILAN® KM50, and the like or anionic surfactants such as dodecylbenzene sulphate, sodium lauryl sulphate or the like. The agents also improve the foaming properties and the dispersion of any precipitate formed in the solution. Selection of these ingredients, which are also detergents, must consider their effect on sticking on tablet punches and on the hardness of the tablet which could increase causing a decrease in the rate of dissolution. The amount of such agents will generally be from 0 to 5% dry weight basis, preferably about 1%. Especially preferred for use in this connection is ricinylmonoethanolamide sulphosuccinate disodium salt sold under the trademark REWO—DERM® S1333 from the firm REWO, Federal Republic of Germany and a mixture of hardened triglycerides material being available in the Federal Republic of Germany under the trademark BOESON®VP 60. Polyethylene glycol, such as for example, PEG 20,000 may also be employed. Generally up to about 3% and preferably about 0.5%, dry weight basis, of the sulfosuccinate acid up to about 5% and preferably about 3%, hardened triglyceride may be employed. However, it is important that the amounts of these ingredients be carefully controlled because the acid layer has a tendency to stick on the tablet punches because of the nature of the sulphamic acid and sodium bisulphate.

It is also desirable to employ an anti-dusting agent to reduce the dust which develops during the manufacture of the bulk powder and during tabletting. An amount of anti-dusting agent up to about 1%, preferably 0.1 to 1% and most preferably about 0.4% by weight. A suitable anti-dusting agent is, for example, light liquid paraffin. However, too high a level of such agent causes a darkening of the color, poor flow properties and reduces binding hence softening tablets with possible capping.

The rate of effervescence and dissolution of the acid layer can also be reduced by the incorporation of magnesium stearate in an amount up to about 1%, dry weight basis, generally 0.1 to 1%, preferably about 0.3%. The magnesium stearate also acts as a lubricating agent to reduce the sticking of the acid layer.

Flavoring agents such as peppermint or other aromatics may be added in an amount of from 0 to 1.5%, preferably about 0.2%. While any suitable dye or colorant could be added in the acid layer it is necessary that a stable dye be employed because of the high level of bleaching agents present in the cleansing solution. The acid layer can, for example, be a green color and this can be accomplished by employing Blue No. 1 Lake and Yellow Tartrazine Lake, for example, in an amount of about 0.15% Blue Lake and 0.2% yellow dye. When different colorings of the two layers is employed whereby for example the first layer is white, and the second layer green, it is possible to optically follow the chronological succession of the two cleansing stages. When a tablet of this type is placed in a glass, or the like, filled with water as a cleansing bath, one can observe that, at the beginning of a cleansing operation, only the first, for example, white layer dissolves rapidly in the cleansing bath with an accompanying turbulent gas-formation without the water being colored thereby. There subsequently follows the dissolution of the second, for example, green layer, connected with a strong but not turbulent gas-formation. The onset of the second cleansing stage is indicated by a clearly evident coloring (or tinting) of the cleansing bath brought about by the dye additive.

Generally the alkaline layer of the two-phase tablet will comprise about 36% of the total weight and the acid layer about 64% although this may vary within the scope of the invention. A suitable tablet may, for example, comprises an alkaline layer of about 1.4 grams and an acid layer of about 2.5 grams.

As exemplary of the tablet formulations of this invention there may be mentioned the following preferred formulation.

| Component | Percent w/w |
|---|---|
| **Alkaline Phase** | |
| Sodium bicarbonate | 10—25 |
| Sodium tripolyphosphate | 4—15 |
| Tetrasodium salt of ethylene diamine tetraacetic acid | 0—5 |
| Sodium perborate monohydrate | 5—40 |
| Potassium monopersulphate | 10—50 |
| Sodium carbonate | 0—15 |
| Ricinylmonoethanolamide sulphsuccinic disodium salt | 0—3.0 |
| Mixture of hardended triglycerides | 0—5.0 |
| Flavorant | 0—1.5 |
| | 100.0 |
| **Acid Phase** | |
| Sulphamic acid | 10—60 |
| Sodium bisulphate | 0—20 |

| | |
|---|---|
| Potassium monopersulphate and/or citric acid | 10—90 |
| Tetrasodium salt of ethylene diamine tetraacetatic acid | 0—10 |
| Sodium carbonate | 0—10 |
| Cetyl pyridinium chloride | 0.1—10 |
| Disodium pyrophosphate | 0—15 |
| Silica | 0—3.0 |
| Ricinylmonoethanolamide sulphosuccinate disodium salt | 0—3.0 |
| Mixture of hardened triglycerides | 0—5.0 |
| Dye or colorant | 0—1.0 |
| Flavorant | 0—1.5 |
| Liquid paraffin | 0—1.0 |
| Magnesium stearate | 0—1.0 |
| | 100.0 |

An especially preferred formulation of this invention is the following formulation.

| Component | Percent w/w |
|---|---|
| **Alkaline White Phase** | |
| Sodium bicarbonate | 17.7 |
| Sodium tripolyphosphate | 8.0 |
| Tetrasodium salt of ethylene diamine tetraacetic acid | 3.0 |
| Sodium perborate monohydrate | 24.0 |
| Potassium monopersulphate | 33.0 |
| Sodium carbonate | 7.0 |
| Ricinylmonoethanolamide sulphosuccinate disodium salt | 1.0 |
| Mixture of hardended triglycerides | 1.0 |
| Peppermint oil | 0.3 |
| Sulphamic acid | 5.0 |
| | 100.0 |
| **Acid Green Phase** | |
| Sulphamic acid | 30.00 |
| Sodium sulphate | 5.00 |
| Potassium monopersulphate or citric acid | 50.10 |

| | |
|---|---|
| Tetrasodium salt of ethylene diamine tetraacetic acid | 2.00 |
| Sodium carbonate | 3.00 |
| Cetyl pyridinium chloride | 2.00 |
| Silicone dioxide | 1.00 |
| Ricinylmonoethanolamide sulphosuccinate disodium salt | 1.00 |
| Mixture of hardended triglycerides | 4.00 |
| Blue No. 1 Lake | 0.15 |
| Yellow Tartrazine Lake | 0.15 |
| Peppermint oil | 0.30 |
| Liquid paraffin | 0.50 |
| Magnesium stearate | 0.80 |
| | 100.0 |

The tablets of this invention are prepared by simply dry mixing the powders for each layer into a homogeneous mixture for each layer and a cleansing tablet comprising a first and second layer is compressed or tabletted from the two resulting respective compositions under controlled humidity of approximately 40% RH.

The anti-plaque activity of a tablet of this invention was tested employing a tablet of the following exemplary formulation.

| Component | Percent w/w |
|---|---|
| **Alkaline Layer** | |
| Potassium monopersulphate | 49.00 |
| Sodium perborate monohydrate | 28.00 |
| Sodium carbonate | 12.50 |
| Sodium tripolyphosphate | 7.00 |
| Polyethyleneglycol (PEG) 10,000 | 2.00 |
| Peppermint powder | 1.50 |
| **Acid Layer** | |
| Citric Acid | 27.00 |
| Sulphamic acid | 24.00 |
| Sodium bisulphate | 7.00 |
| Tetrasodium salt of ethylene diamine diamine tetraacetic acid | 11.80 |
| Sodium perborate monohydrate | 12.00 |
| Potassium monopersulphate | 7.00 |
| Cetylpyridinium chloride | 3.00 |

| | |
|---|---|
| Sodium carbonate | 3.00 |
| PEG 20,000 | 2.00 |
| Peppermint powder | 1.50 |
| Silica | 1.26 |
| Sodium lauryl sulphate | 0.20 |
| Patent Blue V Lake | 0.12 |
| Yellow Tartrazine Lake | 0.12 |

The test method employed to evaluate the anti-plaque activity of the cetylpyridinium chloride containing tablets of this invention was as follows.

Method

1. Subjects

The panel was comprised of 6 senior citizens all of whom had a full set of acrylic dentures. The age of the dentures varied from 2 years to 30 years. They were given no special instructions on treatment of their dentures prior to the trial.

2. Product

Cetylpyridinium chloride tablet of this invention as described above.

3. Test Regime

The top sets of dentures were rinsed with water and then vigorously brushed for 5 minutes with commercial cleanser containing a solution of 6% hydrochloric acid. The dentures were then rinsed with water and disclosed with 5% erythrosine for 10 sec. Excess dye was rinsed off and the plaque coverage was assessed by 4 independent observers. After assessment, the dentures were photographed, using Barfen® CR100 slide film, on the top fitting surface and on both sides, under controlled lighting conditions.

The objective of this treatment was to obtain a baseline measurement of plaque density, as close to zero as possible, against which the effectiveness of subsequent treatments in preventing plaque build-up could be measured.

Residual traces of erythrosine were removed from the dentures by brushing with a 50/50 mixture of sodium lauryl sulphate and sodium perborate. This vigorous treatment was necessary, since the residual plaque stained strongly and the dye proved difficult to remove. After the dye was removed, the dentures were cleansed with the cetylpyridinium chloride (CPC) tablet in pots containing 150 ml of tap water at 37°C for 15 min. The CPC tablets were placed in the water with the white surface uppermost.

The six subjects were given CPC tablets to use between visits. They were asked to clean their dentures every morning and were provided with pots for this purpose. They were also asked to avoid brushing their dentures and the use of any other cleansers. They were permitted to wear their dentures at night if they so desired, but if they chose to soak them, to use only water.

On day 5 the subjects returned and the plaque was assessed, as before, but this time without 6% hydrochlorid acid pre-treatment.

4. Assessment of Plaque

Four observers assessed the plaque according to the area of denture covered, and assigned scores as follows:—

0 — no plaque
1 — Light plaque (1—25% area covered)
2 — moderate plaque (26—50% area covered)
3 — heavy plaque (51—75% area covered)
4 — very heavy plaque (76—100% area covered)

They were asked to visually bisect the top fitting surface of the dentures, horizontally and vertically, and to score the 4 areas separately. Similarly, they bisected each side of the dentures, giving 8 separate areas in total. Thus, the maximum possible score for each denture was 32.

## RESULTS

**Table 1 Total Plaque Scores (Sum of
Scores from 4 Assessors)**

| Subject | Baseline | Treated | Diff. |
|---------|----------|---------|-------|
| 3 | 53 | 44 | −9 |
| 4 | 44 | 57 | +13 |
| 6 | 31 | 29 | −2 |
| 9 | 61 | 38 | −23 |
| 11 | 42 | 53 | +11 |
| 12 | 35 | 41 | +6 |
| Total | 266 | 262 | −4 |

Table 1 shows the combined plaque scores of all 4 assessors for each subject. Since the dentures were cleansed with 6% hydrochloric acid solution on the first visit, and only disclosed on the second visit, we would expect to see an increase in the plaque score over the 4 day treatment period. This should give a measure of the build-up of soft plaque and thus a comparison of the inhibiting effect of these formulations.

In the case of the CPC tablet, the build up of plaque was suppressed, with some subjects actually showing a decrease in plaque index when compared with baseline scores. The overall plaque index was similar after CPC tablet treatment, to the baseline figures. The results show that the CPC containing tablet of this invention is an effective anti-plaque agent.

**Claims**

1. A two layer denture cleansing tablet having anti-plaque activity comprising

(A) a first, faster dissolution, alkaline layer containing active oxygen cleansing and disinfecting compounds which dissolves in water within about two minutes to produce a solution having a pH of about 9.0, and

B) a second, slower dissolution, acid layer containing an anti-plaque effective amount of quaternary ammonium anti-plaque compound selected from

tetradecyl trimethylammonium bromide;

1-hexadecyl(cetyl; pyridinium chloride;

benzyl dimethyl (2-(2-[4-(1,1,3,3-tetra methyl butyl)phenoxy]ethoxy)ethyl)ammonium chloride;

a mixture of alkyl benzyl dimethylammonium chlorides;

dodecyl dimethyl (2-phenoxyethyl)ammonium bromide;

1,1-decamethylene bis (4-amino-2-methylquinolinium chloride); and

dimethyl tetradecyl benzyl ammonium chloride

and acidic compounds which dissolves in water in a period up to about nine minutes and together with the alkaline layer produces a solution having a pH of about $3.0 \pm 0.5$ thereby permitting deposition of an anti-plaque effective amount of the quaternary ammonium compound on dentures.

2. A denture cleansing tablet of claim 1 wherein

A) the alkaline layer comprises, on a dry weight basis based on 100 weight percent total dry weight alkaline layer, a mixture of

(1) from 10 to 25% sodium bicarbonate;

(2) from 4 to 15% sodium tripolyphosphate;

(3) from 5 to 40% sodium perborate, and

(4) from 10 to 50% potassium monopersulphate,

B) the acid layer comprises, on a dry basis based on 100 weight percent total dry weight acid layer, a mixture of

(1) from 10 to 60% sulphamic acid;

(2) from 10 to 90% potassium monopersulphate or citric acid;

(3) from 0.1 to 10% cetylpyridinium chloride.

3. A denture cleansing tablet of claim 2 wherein the hardness of the tablet is in the range of from 6 to 10 kp (58.86 to 98.1 N).

4. A denture cleansing tablet of claim 3 wherein the composition of the alkaline and acid layers is comprised of

| Component | Percent w/w |
|---|---|
| **Alkaline Phase** | |
| Sodium bicarbonate | 10—25 |
| Sodium tripolyphosphate | 4—15 |
| Tetrasodium salt of ethylene diamine tetraacetic acid | 0—5 |
| Sodium perborate monohydrate | 5—40 |
| Potassium monopersulphate | 10—50 |
| Sodium carbonate | 0—15 |
| Ricinylmonoethanolamide sulphsuccinic disodium salt | 0—3.0 |
| Mixture of hardened triglycerides | 0—5.0 |
| Flavorant | 0—1.5 |
| | 100.0 |
| **Acid Phase** | |
| Sulphamic acid | 10—60 |
| Sodium bisulphate | 0—20 |
| Potassium monopersulphate and/or citric acid | 10—90 |
| Tetrasodium salt of ethylene diamine tetraacetic acid | 0—10 |
| Sodium carbonate | 0—10 |
| Cetyl pyridinium chloride | 0.1—10 |
| Disodium pyrophosphate | 0—15 |
| Silica | 0—3.0 |
| Ricinylmonoethanolamide sulphosuccinate disodium salt | 0—3.0 |
| Mixture of hardened triglycerides | 0—5.0 |
| Dye or colorant | 0—1.0 |
| Flavorant | 0—1.5 |
| Liquid paraffin | 0—1.0 |
| Magnesium stearate | 0—1.0 |
| | 100.0 |

5. A denture cleansing tablet of claim 4 wherein the composition of the alkaline and acid layers is comprised of

| Component | Percent w/w |
|---|---|
| **Alkaline White Phase** | |
| Sodium bicarbonate | 17.7 |
| Sodium tripolyphosphate | 8.0 |
| Tetrasodium salt of ethylene diamine tetraacetic acid | 3.0 |
| Sodium perborate monohydrate | 24.0 |
| Potassium monopersulphate | 33.0 |
| Sodium carbonate | 7.0 |
| Ricinylmonoethanolamide sulphosuccinate disodium salt | 1.0 |
| Mixture of hardended triglycerides | 1.0 |
| Peppermint oil | 0.3 |
| Sulphamic acid | 5.0 |
| | 100.0 |
| **Acid Green Phase** | |
| Sulphamic acid | 30.00 |
| Sodium sulphate | 5.00 |
| Potassium monopersulphate or citric acid | 50.10 |
| Tetrasodium salt of ethylene diamine tetraacetic acid | 2.00 |
| Sodium carbonate | 3.00 |
| Cetyl pyridinium chloride | 2.00 |
| Silicon dioxide | 1.00 |
| Ricinylmonoethanolamide sulphosuccinate disodium salt | 1.00 |
| Mixture of hardended triglycerides | 4.00 |
| Blue No. 1 Lake | 0.15 |
| Yellow Tartrazine Lake | 0.15 |
| Peppermint oil | 0.30 |
| Liquid paraffin | 0.50 |
| Magnesium stearate | 0.80 |
| | 100.0 |

11

6. A denture cleansing tablet according to any of claims 1 to 5 wherein the alkaline layer comprises about 36% by weight of the whole tablet and the acid layer comprises about 64% by weight of the whole tablet.

**Patentansprüche**

1. Zweischicht-Zahnreinigungstablette mit Antibelag-Wirksamkeit, umfassend

A) eine erste alkalische Schicht mit schnellerer Auflösung, die reinigende und desinfizierende Verbindungen mit aktivem Sauerstoff enthält, welche sich in Wasser innerhalb von etwa 2 Minuten unter Erzeugung einer Lösung mit einem pH-Wert von etwa 9,0 löst, und

B) eine zweite saure Schicht mit langsamerer Auflösung, die eine gegen Zahnbelag wirksame Menge einer quaternären Ammonium-Antibelag-Verbindung, ausgewählt aus Tetradecyltrimethylammoniumbromid, 1-Hexadecyl (Cetyl)pyridiniumchlorid, Benzyl-dimethyl-(2-(2-[4-(1,1,3,3-tetramethylbutyl)-phenoxy]-äthoxy)-äthyl)-ammmoniumchlorid, einem Gemisch aus Alkylbenzyldimethylammoniumchloriden, Dodecyl-dimethyl-(2-phenoxyäthyl)-ammoniumbromid, 1,1-Decamethylen-bis-(4-amino-2-methyl-chinoliniumchlorid), und Dimethyltetradecylbenzylammoniumchlorid, und saure Verbindungen enthält, welche sich in Wasser in einem Zeitraum bis zu etwa 9 Minuten löst und zusammen mit der alkalischen Schicht eine Lösung mit einem pH-Wert von etwa 3,0 ± 0,5 ergibt und damit die Ablagerung einer gegen Belag wirksamen Menge der quaternären Ammoniumverbindung auf den Zähnen ermöglicht.

2. Zahnreinigungstablette nach Anspruch 1, in der

A) die alkalische Schicht, auf Trockengewichtsbasis, bezogen auf 100 Gew.-% Gesamtgewicht der trockenen alkalischen Schicht, ein Gemisch von

(1) 10 bis 25% Natriumbicarbonat,

(2) 4 bis 15% Natriumtripolyphosphat,

(3) 5 bis 40% Natriumperborat, und

(4) 10 bis 50% Kaliummonopersulfat, umfaßt, und

B) die saure Schicht, auf Trockenbasis, bezogen auf 100 Gew.-% Gesamtgewicht der trockenen sauren Schicht, ein Gemisch von

(1) 10 bis 60% Sulfaminsäure,

(2) 10 bis 90% Kaliummonopersulfat oder Citronensäure, und

(3) 0,1 bis 10% Cetylpyridiniumchlorid, umfaßt.

3. Zahnreinigungstablette nach Anspruch 2, wobei die Härte der Tablette im Bereich von 6 bis 10 kp (58,86 bis 98,1 N) liegt.

4. Zahnreinigungstablette nach Anspruch 3, in der die Zusammensetzung der alkalischen und sauren Schichten umfaßt wird von:

| Komponente | %Gew./Gew. |
|---|---|
| **Alkalische Phase** | |
| Natriumbicarbonat | 10—25 |
| Natriumtripolyphosphat | 4—15 |
| Tetranatriumsalz von Äthylendiamintetraessigsäure | 0—5 |
| Natriumperborat-Monohydrat | 5—40 |
| Kaliummonopersulfat | 10—50 |
| Natriumcarbonat | 0—15 |
| Dinatriumsalz des Ricinylmonoäthanolamids von Sulfobernsteinsäure | 0—3,0 |
| Gemisch von gehärteten Triglyceriden | 0—5,0 |
| Geruchsstoff | 0—1,5 |
| | 100,0 |

**EP 0 151 203 B1**

Saure Phase

| | |
|---|---|
| Sulfaminsäure | 10—60 |
| Natriumbisulfat | 0—20 |
| Kaliummonopersulfat und/oder Citronensäure | 10—90 |
| Tetranatriumsalz von Äthylendiamintetraessigsäure | 0—10 |
| Natriumcarbonat | 0—10 |
| Cetylpyridiniumchlorid | 0,1—10 |
| Dinatriumpyrophosphat | 0—15 |
| Silciumdioxid | 0—3,0 |
| Dinatriumsalz des Ricinylmonoäthanolamids von Sulfobernsteinsäure | 0—3,0 |
| Gemisch von gehärteten Triglyceriden | 0—5,0 |
| Farbstoff oder Färbestoff | 0—1,0 |
| Geruchsstoff | 0—1,5 |
| Flüssiges Paraffin | 0—1,0 |
| Magnesiumstearat | 0—1,0 |
| | 100 |

5. Zahnreinigungstablette nach Anspruch 4, in der die Zusammensetzung der alkalischen und sauren Schichten umfaßt wird von:

| Komponente | %Gew./Gew. |
|---|---|
| Alkalische weiße Phase | |
| Natriumbicarbonat | 17,7 |
| Natriumtripolyphosphat | 8,0 |
| Tetranatriumsalz von Äthylendiamintetraessigsäure | 3,0 |
| Natriumperborat-Monohydrat | 24,0 |
| Kaliummonopersulfat | 33,0 |
| Natriumcarbonat | 7,0 |
| Dinatriumsalz des Ricinylmonoäthanolamids von Sulfobernsteinsäure | 1,0 |
| Gemisch von gehärteten Triglyceriden | 1,0 |
| Pfefferminzöl | 0,3 |
| Sulfaminsäure | 5,0 |
| | 100 |

# EP 0 151 203 B1

| Saure grüne Phase | |
|---|---|
| Sulfaminsäure | 30 |
| Natriumsulfat | 5,0 |
| Kaliummonopersulfat oder Citronensäure | 50,10 |
| Tetranatriumsalz von Äthylendiamintetraessigsäure | 2,0 |
| Natriumcarbonat | 3,0 |
| Cetylpyridiniumchlorid | 2,0 |
| Siliciumdioxid | 1,0 |
| Dinatriumsalz des Ricinylmonoäthanolamids der Sulfobernsteinsäure | 1,0 |
| Gemisch von gehärteten Triglyceriden | 4,00 |
| Farbstoff Blue Nr. 1 | 0,15 |
| Farbstoff Yellow Tartrazine | 0,15 |
| Pfefferminzöl | 0,30 |
| Flüssiges Paraffin | 0,50 |
| Magnesiumstearat | 0,80 |
| | 100,0 |

6. Zahnreinigungstablette nach einem der Ansprüche 1 bis 5, in der die alkalische Schicht etwa 36 Gew.-% der gesamten Tablette und die saure Schicht etwa 64 Gew.-% der gesamten Tablette umfaßt.

## Revendications

1. Comprimé de nettoyage pour prothèses dentaires à deux couches ayant une activité anti-plaque comprenant

A) une première couche alcaline, de dissolution plus rapide, contenant des composés de nettoyage et de désinfection à oxygène actif, qui se dissout dans l'eau après environ deux minutes pour produire une solution ayant un pH d'environ 9,0, et

B) une seconde couche acide, de dissolution plus lente, contenant une quantité anti-plaque efficace de composé anti-plaque d'ammonium quaternaire choisi parmi le bromure de tétradécyl triméthyl-ammonium, le chlorure de l'hexadécyl (cétyl) pyridinium, le chlorure de benzyl diméthyl (2-(2-[4-(1,1,3,3-tétra méthyl butyl)phénoxy]éthoxy)éthyl)ammonium, les mélanges de chlorures d'alkyl benzyl diméthyl-ammonium, le bromure de dodécyl diméthyl (2-phénoxyéthyl)ammonium, le 1,1-décaméthylène bis (chlorure de 4-amino-2-méthylquinoléinium) et le chlorure de diméthyl tétradécyl benzyl ammonium, et des composés acides, qui se dissout dans l'eau en une période atteignant environ neuf minutes et en même temps que la couche alcaline produit une solution ayant un pH d'environ 3,0 ± 0,5, en permettant ainsi le dépôt d'une quantité anti-plaque efficace du composé d'ammonium quaternaire sur les prothèses dentaires.

2. Comprimé de nettoyage pour prothèses dentaires suivant la revendication 1, caractérisé en ce que:

A) la couche alcaline comprend, sur une base pondérale sèche, basée sur un poids sec total de 100% en poids de couche alcaline, un mélange:
    (1) de 10 à 25% de bicarbonate de sodium;
    (2) de 4 à 15% de tripolyphosphate de sodium;
    (3) de 5 à 40% de perborate de sodium; et
    (4) de 10 à 50% de monopersulfate de potassium,

B) la couche acide comprend, sur une base sèche basée sur un poids sec total de 100% en poids de couche acide, un mélange
    (1) de 10 à 60% d'acide sulfamique;
    (2) de 10 à 90% de monopersulfate de potassium ou d'acide citrique;
    (3) de 0,1 à 10% de chlorure de cétylpyridinium.

14

3. Comprimé de nettoyage pour prothèses dentaires suivant la revendication 2, caractérisé en ce que la dureté du comprimé est de l'ordre de 6 à 10 kp (58,86 à 98,1 N).

4. Comprimé de nettoyage pour prothèses dentaires suivant la revendication 3, caractérisé en ce que la composition des couches alcaline et acide est formée de

| Composant | % en poids/poids |
|---|---|
| **Phase alcaline** | |
| Bicarbonate de sodium | 10—25 |
| Tripolyphosphate de sodium | 4—15 |
| Sel tétrasodique d'acide éthylène diamine tétraacétique | 0—5 |
| Perborate de sodium monohydraté | 5—40 |
| Monopersulfate de potassium | 10—50 |
| Carbonate de sodium | 0—15 |
| Sel disodique sulfosuccinique de ricinylmonoéthanolamide | 0—3,0 |
| Mélange de triglycérides durcis | 0—5,0 |
| Aromatisant | 0—1,5 |
| | 100,0 |
| **Phase acide** | |
| Acide sulfamique | 10—60 |
| Bisulfate de sodium | 0—20 |
| Monopersulfate de potassium et/ou acide citrique | 10—90 |
| Sel tétrasodique d'acide éthylène diamine tétraacétatique | 0—10 |
| Carbonate de sodium | 0—10 |
| Chloururure de cétylpyridinium | 0,1—10 |
| Pyrophosphate disodique | 0—15 |
| Silice | 0—3,0 |
| Sel disodique de sulfosuccinate de ricinylmonoéthanolamide | 0—3,0 |
| Mélange de triglycérides durcis | 0—5,0 |
| Teinture ou colorant | 0—1,0 |
| Aromatisant | 0—1,5 |
| Paraffine liquide | 0—1,0 |
| Stéarate de magnésium | 0—1,0 |
| | 100,0 |

5. Comprimé de nettoyage pour prothèses dentaires suivant la revendication 4, caractérisé en ce que la composition des couches alcaline et acide est formée de:

| Composant | % en poids/poids |
|---|---|
| **Phase alcaline blanche** | |
| Bicarbonate de sodium | 17,7 |
| Tripolyphosphate de sodium | 8,0 |
| Sel tétrasodique d'acide éthylène diamine tétraacétique | 3,0 |
| Perborate de sodium monohydraté | 24,0 |
| Monopersulfate de potassium | 33,0 |
| Carbonate de sodium | 7,0 |
| Sel disodique de sulfosuccinate de ricinylmonoéthanolamide | 1,0 |
| Mélange de triglycérides durcis | 1,0 |
| Essence de menthe poivrée | 0,3 |
| Acide sulfamique | 5,0 |
| | 100,0 |
| **Phase acide verte** | |
| Acide sulfamique | 30,00 |
| Sulfate de sodium | 5,00 |
| Monopersulfate de potassium ou acide citrique | 50,10 |
| Sel tétrasodique d'acide éthylène diamine tétraacétique | 2,00 |
| Carbonate de sodium | 3,00 |
| Chlorure de cétyl pyridinium | 2,00 |
| Dioxyde de silicium | 1,00 |
| Sel disodique de sulfosuccinate de ricinylmonoéthanolamide | 1,00 |
| Mélange de triglycérides durcis | 4,00 |
| Laque bleue n° 1 | 0,15 |
| Laque de tartrazine jaune | 0,15 |
| Essence de menthe poivrée | 0,30 |
| Paraffine liquide | 0,50 |
| Stéarate de magnésium | 0,80 |
| | 100,0 |

6. Comprimé de nettoyage pour prothèse dentaire suivant l'une quelconque des revendications 1 à 5, caractérisé en ce que la couche alcaline constitue environ 36% en poids du comprimé total et la couche acide constitue environ 64% en poids du comprimé total.